# EUROPÄISCHE PATENTANMELDUNG

(11) **EP 1 698 288 A2**
(43) Veröffentlichungstag der Anmeldung: **06.09.2006**
(21) Anmeldenummer: 06110505.2
(22) Anmeldetag: 28.02.2006
(51) Int. Cl.: A61B 17/22

(54) **Stoßwellensystem**

(30) Priorität: 01.03.2005 DE 102005009907
(71) Anmelder: SIEMENS AKTIENGESELLSCHAFT, 80333 München (DE)
(72) Erfinder: Lanski, Markus, 90408 Nürnberg (DE); Meinert, Christian, 91080 Marloffstein (DE)

(57) **Zusammenfassung**

Die Erfindung betrifft ein Stoßwellensystem mit einer Stoßwellenquelle, die Stoßwellen für eine Behandlung eines Patienten erzeugt, wobei nach einer Unterbrechung der Behandlung die Stoßwellenenergie, ausgehend von einem vorgebbaren Energie-Anfangswert, automatisch in vorgebbaren Energiestufen sukzessive auf einen letzten vor der Unterbrechung der Be-handlung applizierten und gespeicherten Energie-Endwert erhöht wird. Ein derartiges Stoßwellensystem ermöglicht nach einer Behandlungsunterbrechung eine optimierte Wiederaufnahme der Stoßwellenbehandlung.

## Beschreibung

Die Erfindung betrifft ein Stoßwellensystem mit einer Stoßwellenquelle, die Stoßwellen für eine Behandlung eines Patienten erzeugt.

Ein derartiges Stoßwellensystem dient zur Behandlung mit extrakorporalen Stoßwellen, meistens in der Lithotripsie und in der Schmerztherapie, insbesondere in der extrakorporalen Stoßwellentherapie (ESWT).

Bei der Lithotripsie handelt es sich um eine therapeutische Methode, ein im Körper eines Lebewesens befindliches Konkrement (z.B. Gallenstein, Nierenstein) ohne operativen Eingriff mit Hilfe von fokussierten Stoßwellen zu zerstören. Sowohl in der Lithotripsie als auch in der extrakorporalen Stoßwellentherapie wird zu Beginn der Behandlung mit niedrigen Energiewerten begonnen, um den Patienten an die Behandlung und die damit möglicherweise verbundenen Schmerzen zu gewöhnen. Dieses langsame Erhöhen der Energiewerte wird als Ramping bezeichnet. Ziel ist es, für die jeweilige Applikation eine möglichst hohe Energiestufe zu erreichen, um eine effektive Therapie zu gewährleisten. Wird nun die Stoßwellenapplikation am Patienten aus verschiedenen Gründen für einen Zeitraum von mehr als ca. einer Minute unterbrochen, so wird der Patient von der Stoßwelle "entwöhnt". Bei einer Wiederaufnahme der Therapie muss der Patient deshalb erneut mittels Ramping an die Stoßwelle gewöhnt werden. Dieses Ramping wird vom Anwender manuell durchgeführt, bis der letzte applizierte Energiewert der Stoßwelle erreicht ist.

Der Erfindung liegt nun die Aufgabe zugrunde, ein Stoßwellensystem der eingangs genannten Art zu schaffen, das nach einer Behandlungsunterbrechung eine optimierte Wiederaufnahme der Stoßwellenbehandlung ermöglicht.

Diese Aufgabe wird erfindungsgemäß durch ein Stoßwellensystem mit den Merkmalen des Anspruchs 1 gelöst. Vorteilhafte Ausgestaltungen der Erfindung sind jeweils Gegenstand von weiteren Ansprüchen.

Das Stoßwellensystem nach Anspruch 1 umfasst eine Stoßwellenquelle, die Stoßwellen für eine Behandlung eines Patienten erzeugt, wobei nach einer Unterbrechung der Behandlung die Stoßwellenenergie, ausgehend von einem vorgebbaren Energie-Anfangswert, automatisch in vorgebbaren Energiestufen sukzessive auf einen letzten vor der Unterbrechung der Behandlung applizierten und gespeicherten Energie-Endwertwert erhöht wird.

Das Stoßwellensystem gemäß Anspruch 1 ermöglicht nach einer Behandlungsunterbrechung eine optimierte Wiederaufnahme der Stoßwellenbehandlung beim Patienten. Darüber hinaus können die Energiestufen wesentlich feiner als bei einer manuellen Erhöhung ausgeprägt sein. Hierzu wird bei jeder einzelnen Stoßwelle die Hochspannung, welche den Energiewert bestimmt, nur um einen sehr kleinen Betrag erhöht. Damit sind Änderungen möglich, die nur einem Bruchteil einer konventionellen (manuellen) Erhöhung der Energiewerte entsprechen. Dadurch ist eine sehr schonende Gewöhnung des Patienten an den letzten applizierten Energie-Endwert möglich, da ein sprungweises Erhöhen der Energiewerte sowie ein damit verbundenes starkes Ansteigen des Schmerzempfindens zuverlässig vermieden wird.

Da die Erhöhung auf den letzten vor der Unterbrechung der Behandlung applizierten Energie-Endwertwert automatisch erfolgt, müssen vom Anwender lediglich die Parameter für die Energiestufen vorgegeben werden (dies kann z.B. bei der Inbetriebnahme des Stoßwellensystems erfolgen) und das Stoßwellensystem bei einem vorgebbaren Energie-Anfangswert gestartet werden. Weitere Eingriffe des Anwenders sind bis zum Erreichen des Energie-Endwertes in der Regel nicht mehr erforderlich.

Die Erhöhung der Stoßwellenenergie ist im Rahmen der Erfindung in vielfältiger Weise parametrierbar. So kann z.B. gemäß Anspruch 2 der vorgebbare Energie-Anfangswert, bei dem die Behandlung nach einer Unterbrechung wieder aufgenommen wird, konstant, insbesondere Null, sein.

Gemäß einer weiteren Ausführungsform nach Anspruch 3 ist der Energie-Anfangswert in Abhängigkeit von dem vor der letzten Unterbrechung der Behandlung applizierten Energie-Endwert vorgebbar. Damit kann - falls beim Patienten bereits eine gewisse Gewöhnung an die Stoßwellen erreicht wurde - mit einem höheren Energie-Anfangswert (Einsprungswert) als dem minimalen Energie-Anfangswert begonnen werden. Die Zeit bis zum Erreichen des vor der Unterbrechung der Behandlung applizierten Energie-Endwertes kann dadurch deutlich verkürzt werden.

Bei einem Stoßwellensystem gemäß Anspruch 4 ist die sukzessive Erhöhung der Stoßwellenenergie derart feinstufig durchführbar, dass die Erhöhung im Wesentlichen einen stetigen Verlauf aufweist. Je feinstufiger die Erhöhung der Stoßwellenenergie durchgeführt wird, desto schonender ist die Gewöhnung des Patienten an den letzten vor der Unterbrechung der Behandlung applizierten Energie-Endwert möglich.

Dieser Verlauf der Erhöhung der Stoßwellenenergie kann hierbei, wie in Anspruch 5 angegeben, im Wesentlichen eine Gerade bilden. Im Rahmen der Erfindung sind bei der Erhöhung der Stoßwellenenergie jedoch auch andere, von einer Geraden abweichende Verläufe möglich.

Ist der Verlauf der Erhöhung der Stoßwellenenergie - wie bei einer Ausgestaltung nach Anspruch 6 - durch eine vorgebbare Funktion definiert, dann kann gemäß Anspruch 7 diese Funktion beispielsweise in Abhängigkeit von dem vor der letzten Unterbrechung der Behandlung applizierten Energie-Endwert und/oder in Abhängigkeit von einer Funktion des Verlaufs einer vorherigen Erhöhung der Stoßwellenenergie vorgegeben werden.

Bei einer vorteilhaften Ausführungsform der Stoßwellensystems nach Anspruch 8 schaltet die automatische Erhöhung der Stoßwellenenergie bei einem manuellen Eingriff ab und wechselt in einen manuellen Modus.

Weiterhin ist es im Rahmen der Erfindung möglich, dass - wie in Anspruch 9 beschrieben - die Stoßwellenenergie nach dem Erreichen des vor der letzten Unterbrechung der Behandlung applizierten Energie-Endwertes manuell auf einen neuen Energie-Endwert einstellbar ist.

Eine besonders benutzerfreundliche Variante des erfindungsgemäßen Stoßwellensystems stellen die Ausgestaltungen nach den Ansprüchen 10 und 11 dar, bei denen die Werte und/oder die Zwischenwerte der Energiestufen anzeigbar sind, bzw. der Verlauf der Erhöhung der Stoßwellenenergie und/oder Verlauf der Zwischenwerte grafisch darstellbar ist.

Durch die vorgenannten Parametrierungsmöglichkeiten lässt sich der Verlauf der Erhöhung der Stoßwellenenergie auf einfache Weise an unterschiedliche Behandlungsmethoden und an das unterschiedliche Schmerzempfinden einzelner Patienten anpassen. Auch anwenderspezifische Applikationsvarianten sind damit einfach realisierbar. Diese Parametrisierung kann sowohl einmalig durch eine Serviceeinstellung bei der Erstinbetriebnahme bzw. bei Wartungsarbeiten als auch durch den Anwender selbst erfolgen.

Nachfolgend wird anhand von zwei verschiedenen Einstellungen des erfindungsgemäßen Stoßwellensystems die Erfindung näher erläutert. Es zeigen:
- FIG 1: einen Verlauf der Erhöhung der Stoßwellenenergie gemäß einer ersten Parametrierung,
- FIG 2: einen Verlauf der Erhöhung der Stoßwellenenergie gemäß einer zweiten Parametrierung.

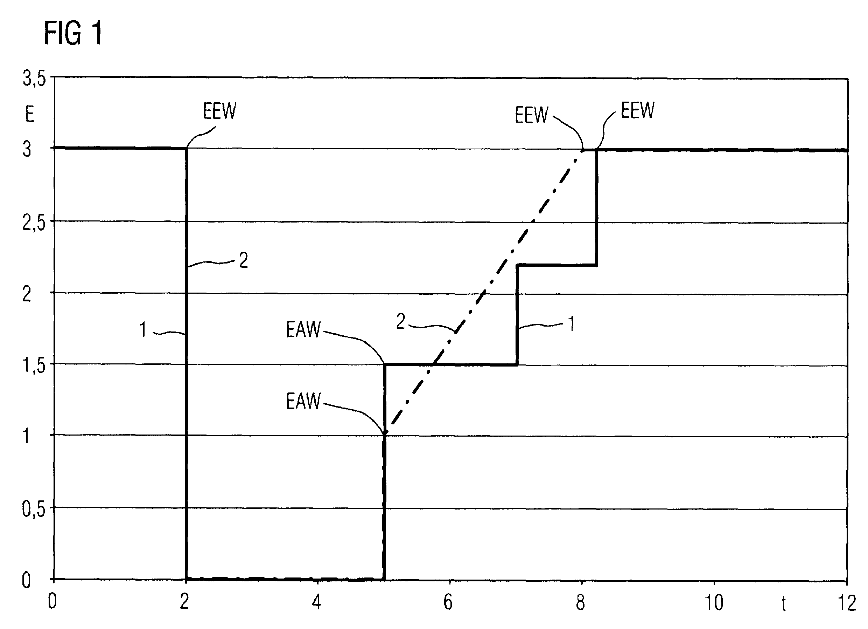

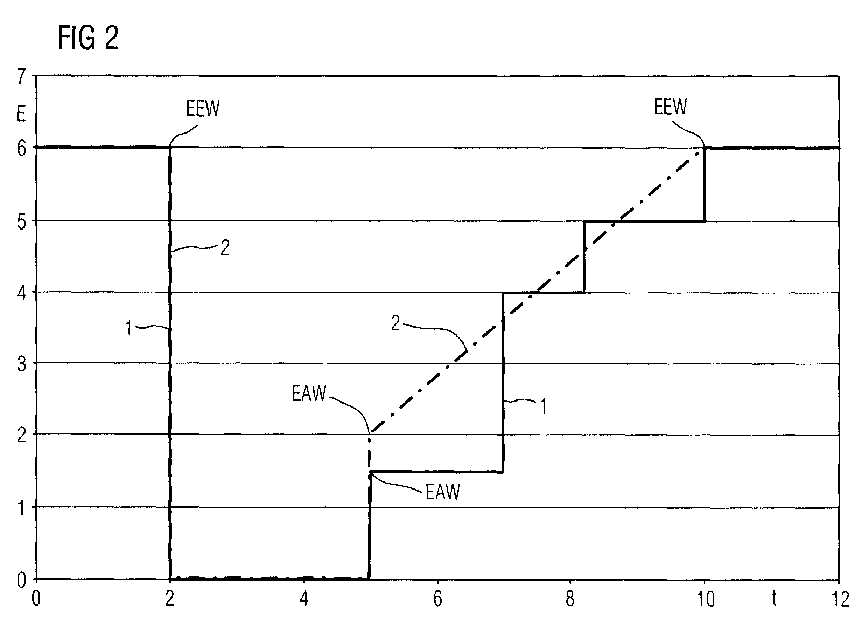

In den FIG 1 und 2 ist auf der Abszissenachse jeweils die Zeit t als dimensionslose Größe aufgetragen. Weiterhin ist auf der Ordinatenachse jeweils die Stoßwellenenergie E als dimensionslose Größe aufgetragen.

In FIG 1 und 2 ist ein möglicher Verlauf einer manuellen Erhöhung der Stoßwellenenergie E mit 1 bezeichnet. Ein mit dem erfindungsgemäßen Stoßwellensystem möglicher Verlauf einer automatischen Erhöhung der Stoßwellenenergie E ist mit 2 bezeichnet.

Die in FIG 1 dargestellte Behandlung wird bei einem Energie-Endwert EEW = 3 bei t = 2 manuell unterbrochen. Bei t = 5 wird die Behandlung mit einem Energie-Anfangswert EAW = 1,5 fortgesetzt.

Die Energiestufen weisen unterschiedliche Höhen sowie unterschiedliche Breiten auf. Bei etwa t = 8,1 ist mit dem Energie-Endwert EEW = 3 der vor der letzten Unterbrechung der Behandlung applizierte Energie-Endwert erreicht.

Gegenüber der manuellen Erhöhung der Stoßwellenenergie erfolgt die automatische Erhöhung der Stoßwellenenergie bei dem erfindungsgemäßen Stoßwellensystem sukzessive und derart feinstufig, dass die Erhöhung im Wesentlichen einen stetigen Verlauf aufweist. Im dargestellten Ausführungsbeispiel bildet der Verlauf der Erhöhung der Stoßwellenenergie im Wesentlichen eine Gerade.

Der gewählte Verlauf beginnt mit einem Energie-Anfangswert, der zum Zeitpunkt t = 5 bei EAW = 1 liegt und etwa eine Steigung von 0,67 aufweist. Der Energie-Endwert EEW = 3 wird bei t = 8 erreicht.

Die in FIG 2 dargestellte Behandlung wird bei einem Energie-Endwert EEW = 6 bei t = 2 manuell unterbrochen. Bei t = 5 wird die Behandlung mit einem Energie-Anfangswert EAW = 1,5 fortgesetzt.

Die Energiestufen weisen unterschiedliche Höhen sowie unterschiedliche Breiten auf. Bei t = 10 ist mit dem Energie-Endwert EEW = 6 der vor der letzten Unterbrechung der Behandlung applizierte Energie-Endwert erreicht.

Gegenüber der manuellen Erhöhung der Stoßwellenenergie erfolgt die automatische Erhöhung der Stoßwellenenergie wiederum sukzessive und derart feinstufig, dass die Erhöhung im Wesentlichen einen stetigen Verlauf aufweist. Im dargestellten Ausführungsbeispiel bildet der Verlauf der Erhöhung der Stoßwellenenergie ebenfalls im Wesentlichen eine Gerade.

Der gewählte Verlauf beginnt mit einem Energie-Anfangswert, der zum Zeitpunkt t = 5 bei EAW = 2 liegt und etwa eine Steigung von 0,8 aufweist. Der Energie-Endwert EEW = 6 wird bei t = 10 erreicht.

Aus der Erläuterung der beiden nicht einschränkenden Ausführungsbeispiele der Erfindung wird deutlich, dass mit dem erfindungsgemäßen Stoßwellensystem nach einer Behandlungsunterbrechung auf einfache Weise eine optimierte Wiederaufnahme der Stoßwellenbehandlung möglich ist.

## Patentansprüche

1. Stoßwellensystem mit einer Stoßwellenquelle, die Stoßwellen für eine Behandlung eines Patienten erzeugt, wobei nach einer Unterbrechung der Behandlung die Stoßwellenenergie, ausgehend von einem vorgebbaren Energie-Anfangswert, automatisch in vorgebbaren Energiestufen sukzessive auf einen letzten vor der Unterbrechung der Behandlung applizierten und gespeicherten Energie-Endwert erhöht wird.

2. Stoßwellensystem nach Anspruch 1, wobei der Energie-Anfangswert konstant, insbesondere Null, ist.

3. Stoßwellensystem nach Anspruch 1, wobei der Energie-Anfangswert in Abhängigkeit von dem vor der letzten Unterbrechung der Behandlung applizierten Energie-Endwert vorgebbar ist.

4. Stoßwellensystem nach Anspruch 1, wobei die sukzessive Erhöhung der Stoßwellenenergie derart feinstufig durchführbar ist, dass die Erhöhung im Wesentlichen einen stetigen Verlauf aufweist.

5. Stoßwellensystem nach Anspruch 4, wobei der Verlauf der Erhöhung der Stoßwellenenergie im Wesentlichen eine Gerade bildet.

6. Stoßwellensystem nach Anspruch 3 oder 4, wobei der Verlauf der Erhöhung der Stoßwellenenergie durch eine vorgebbare Funktion definiert ist.

7. Stoßwellensystem nach Anspruch 6, wobei die Funktion in Abhängigkeit von dem vor der letzten Unterbrechung der Behandlung applizierten Energie-Endwert und/oder in Abhängigkeit von einer Funktion des Verlaufs einer vorherigen Erhöhung der Stoßwellenenergie vorgebbar ist.

8. Stoßwellensystem nach Anspruch 1, wobei die automatische Erhöhung der Stoßwellenenergie bei einem manuellen Eingriff abschaltet und in einen manuellen Modus wechselt.

9. Stoßwellensystem nach Anspruch 1, wobei die Stoßwellenenergie nach dem Erreichen des vor der letzten Unterbrechung der Behandlung applizierten Energie-Endwertes manuell auf einen neuen Energie-Endwert einstellbar ist.

10. Stoßwellensystem nach Anspruch 1, wobei die Werte und/oder die Zwischenwerte der Energiestufen anzeigbar sind.

11. Stoßwellensystem nach Anspruch 1, wobei der Verlauf der Erhöhung der Stoßwellenenergie und/oder Verlauf der Zwischenwerte grafisch darstellbar ist.
